# EUROPEAN PATENT APPLICATION

(11) **EP 4 283 738 A1**
(43) Date of publication of application: **29.11.2023**
(21) Application number: 22742519.6
(22) Date of filing: 14.01.2022
(51) Int. Cl.: H01M 10/0567, C07C 307/00, H01M 10/052, H01M 10/054, H01M 10/0569

(54) **NONAQUEOUS ELECTROLYTE SOLUTION, NONAQUEOUS ELECTROLYTE BATTERY AND COMPOUND**

(30) Priority: 22.01.2021 JP 2021009171
(71) Applicant: Central Glass Co., Ltd., Yamaguchi 755-0001 (JP)
(72) Inventor: TERADA Ryosuke, Ube-shi, Yamaguchi 755-0001 (JP); KAWABATA Wataru, Ube-shi, Yamaguchi 755-0001 (JP); MORI Katsumasa, Ube-shi, Yamaguchi 755-0001 (JP); MORINAKA Takayoshi, Ube-shi, Yamaguchi 755-0001 (JP); TAKAHASHI Mikihiro, Ube-shi, Yamaguchi 755-0001 (JP)
(74) Representative: Mewburn Ellis LLP
(86) International application number: PCT/JP2022/001223
(87) International publication number: WO 2022/158397

(57) **Abstract**

According to a nonaqueous electrolytic solution containing: a compound represented by the General Formula (1); a solute; and a nonaqueous organic solvent, a nonaqueous electrolytic solution battery, and a compound represented by General Formula (1), a nonaqueous electrolytic solution and a nonaqueous electrolytic solution battery having a low initial resistance value, and a compound that can be suitably used for the above nonaqueous electrolytic solution are provided, 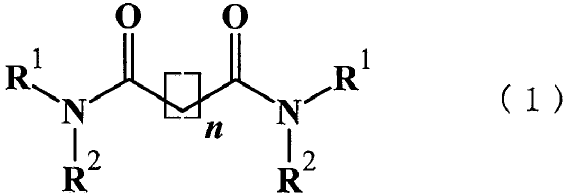 in the General Formula (1), R¹'s each independently represent PO(R_{f})₂ or SO₂R_{f}, R_{f}'s each independently represent a fluorine atom, a linear alkyl group having 1 to 4 carbon atoms, or a branched alkyl group having 3 to 4 carbon atoms, and an oxygen atom may be included between a carbon atom-carbon atom bond in the alkyl group, any hydrogen atom of the alkyl group may be substituted with a fluorine atom, R²'s each independently represent a hydrogen atom, a lithium ion, a sodium ion, a potassium ion, a linear alkyl group having 1 to 12 carbon atoms, or a branched alkyl group having 3 to 12 carbon atoms, and an oxygen atom may be included between a carbon atom-carbon atom bond in the alkyl group, any hydrogen atom of the alkyl group may be substituted with a fluorine atom, the alkyl group may contain an unsaturated bond, when R² represents a lithium ion, a sodium ion, or a potassium ion, a bond between a nitrogen atom and R² in the General Formula (1) represents an ionic bond, and n represents an integer of 0 to 3.

## Description

### TECHNICAL FIELD

The present disclosure relates to a nonaqueous electrolytic solution, a nonaqueous electrolytic solution battery, and a compound.

### BACKGROUND ART

As measures for improving cycle characteristics, high temperature storability, and durability of a nonaqueous electrolytic solution battery, optimization of various constituting elements of the battery including active materials of a positive electrode and a negative electrode has been hitherto studied. Techniques relating to a nonaqueous electrolytic solution are also no exception, in which it has been proposed to use a variety of additives to prevent deterioration of an electrolytic solution at surfaces of active positive and negative electrodes due to decomposition of the electrolytic solution. For example, Patent Literature 1 proposes that various battery characteristics such as high temperature storage characteristics are improved by adding vinylene carbonate to an electrolytic solution. This method is for preventing the electrolytic solution from decomposing on an electrode surface by coating an electrode with a polymer film formed by polymerization of vinylene carbonate, whose problem is that lithium ions also have difficulty in passing through the film so that internal resistance is increased and a lot of gas is generated during high temperature storage. Addition of lithium difluorophosphate disclosed in Patent Literature 2 is effective to solve this problem, and it is known that by using vinylene carbonate and lithium difluorophosphate together, a battery can be obtained in which an increase in internal resistance and generation of gas are prevented while maintaining excellent high temperature storage characteristics.

In addition, Patent Literature 3 discloses a method for improving an initial charge capacity, input and output characteristics, and impedance characteristics by including a compound having fluorosulfonate in an electrolytic solution as a single additive instead of a combination of a plurality of additives.

### CITATION LIST

### PATENT LITERATURE

Patent Literature 1: JP3438636B
Patent Literature 2: JP3439085B
Patent Literature 3: JP2013-152956A

### SUMMARY OF INVENTION

### TECHNICAL PROBLEM

However, as a result of studies by the present inventors, it was found that even if lithium difluorophosphate is added to a nonaqueous electrolytic solution containing vinylene carbonate, an effect of preventing an increase in internal resistance is small, and even if a lithium fluorosulfonate-containing nonaqueous electrolytic solution disclosed in Patent Literature 3 is used, an effect of improving initial input and output characteristics is small. Thus, there is room for improvement in resistance characteristics, particularly in initial resistance characteristics.

The present disclosure is made in view of the above circumstances, and an object thereof is to provide a nonaqueous electrolytic solution and a nonaqueous electrolytic solution battery which have a low initial resistance value. Another object is to provide a compound that can be suitably used in the above nonaqueous electrolytic solution.

### SOLUTION TO PROBLEM

In view of the problem, the present inventors have conducted extensive research and found that a nonaqueous electrolytic solution battery having a low initial resistance value is obtained by a nonaqueous electrolytic solution containing a compound represented by General Formula (1), a solute, and a nonaqueous organic solvent.

That is, the inventors have found that the above problem can be solved by the following configurations.
[1] A nonaqueous electrolytic solution, containing: a compound represented by the following General Formula (1); a solute; and a nonaqueous organic solvent. 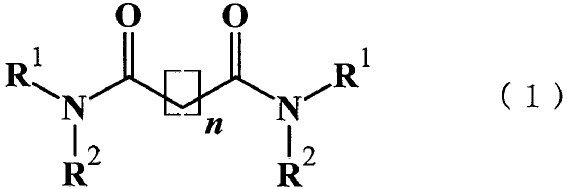 [In General Formula (1), R¹'s each independently represent PO(R_{f})₂ or SO₂R_{f}, R_{f}'s each independently represent a fluorine atom, a linear alkyl group having 1 to 4 carbon atoms, or a branched alkyl group having 3 to 4 carbon atoms, an oxygen atom may be included between a carbon atom-carbon atom bond in the alkyl group, and any hydrogen atom of the alkyl group may be substituted with a fluorine atom. R²'s each independently represent a hydrogen atom, a lithium ion, a sodium ion, a potassium ion, a linear alkyl group having 1 to 12 carbon atoms, or a branched alkyl group having 3 to 12 carbon atoms, and an oxygen atom may be included between a carbon atom-carbon atom bond in the alkyl group, any hydrogen atom of the alkyl group may be substituted with a fluorine atom, and the alkyl group may contain an unsaturated bond. When R² represents a lithium ion, a sodium ion, or a potassium ion, a bond between a nitrogen atom and R² in General Formula (1) represents an ionic bond, and n represents an integer of 0 to 3.]
[2] The nonaqueous electrolytic solution according to [1], in which R¹'s in General Formula (1) each independently represent POF₂ or SO₂F.
[3] The nonaqueous electrolytic solution according to [1] or [2], in which both R¹'s in General Formula (1) are SO₂F.
[4] The nonaqueous electrolytic solution according to any one of [1] to [3], in which R²'s in General Formula (1) each independently represent a hydrogen atom, a lithium ion, a sodium ion, a linear alkyl group having 1 to 4 carbon atoms, or a branched alkyl group having 3 to 4 carbon atoms.
[5] The nonaqueous electrolytic solution according to any one of [1] to [4], in which the compound represented by General Formula (1) is a compound represented by the following Formula (1a). 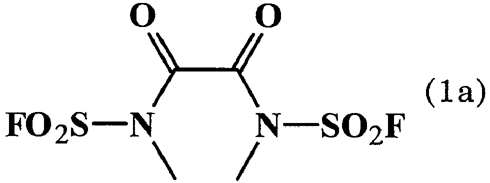
[6] The nonaqueous electrolytic solution according to any one of [1] to [5], in which the nonaqueous organic solvent contains at least one selected from the group consisting of cyclic carbonate and chain carbonate.
[7] The nonaqueous electrolytic solution according to [6], in which the cyclic carbonate is at least one selected from the group consisting of ethylene carbonate, propylene carbonate, and fluoroethylene carbonate, and the chain carbonate is at least one selected from the group consisting of ethyl methyl carbonate, dimethyl carbonate, diethyl carbonate, and methyl propyl carbonate.
[8] The nonaqueous electrolytic solution according to any one of [1] to [7], in which a content of the compound represented by General Formula (1) with respect to a total amount of the compound represented by General Formula (1), the solute, and the nonaqueous organic solvent is 0.001% by mass to 10.0% by mass.
[9] The nonaqueous electrolytic solution according to any one of [1] to [8], further contains at least one selected from vinylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, and lithium (difluorophosphoryl)(fluorosulfonyl)imide in an amount of 0.01% by mass to 5.0% by mass with respect to a total amount of the nonaqueous electrolytic solution.
[10] The nonaqueous electrolytic solution according to any one of [1] to [9], further contains at least one compound selected from the group consisting of compounds represented by the following General Formulas (2) to (3) is contained in an amount of 0.01% by mass to 5.0% by mass with respect to a total amount of the nonaqueous electrolytic solution.

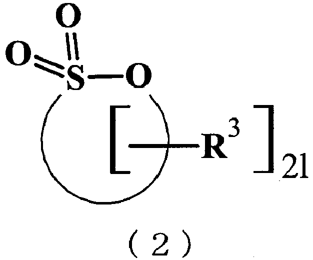

[General Formula (2) represent a compound having a cyclic skeleton composed of 4 to 7 atoms selected from any of carbon atoms, oxygen atoms, and sulfur atoms, and has at least one adjacent sulfonyl group and oxygen atom in the cyclic skeleton, the cyclic skeleton may contain an unsaturated bond, a state of the sulfur atom forming the cyclic skeleton is selected from any of S, SO, and SO₂, l represents the number of carbon atoms contained in the cyclic skeleton, R³'s are bonded to the carbon atom forming the cyclic skeleton and each independently represent a hydrogen atom, a fluorine atom, or an aliphatic hydrocarbon group having 1 to 6 carbon atoms, and the aliphatic hydrocarbon group may contain at least one of an oxygen atom and an unsaturated bond, and any hydrogen atom in the aliphatic hydrocarbon group may be substituted with a fluorine atom, when the carbon atoms forming the cyclic skeleton are adjacent to each other, these carbon atoms may be bonded to form an aromatic ring, and a hydrocarbon group having 1 to 4 carbon atoms may be bonded to the aromatic ring, the hydrocarbon group bonded to the aromatic ring may contain at least one of an oxygen atom and an unsaturated bond, and any hydrogen atom of the hydrocarbon group may be substituted with a fluorine atom.]

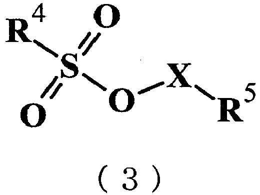

[In General Formula (3), X represents a sulfonyl group or a ketone group, and R⁴ and R⁵ are each independently selected from aliphatic hydrocarbon groups having 1 to 10 carbon atoms. At least one selected from the group consisting of a fluorine atom, an oxygen atom, an unsaturated bond, and an ester bond may be present in the aliphatic hydrocarbon group.]

[11] A nonaqueous electrolytic solution battery, including: a positive electrode; a negative electrode; and the nonaqueous electrolytic solution according to any one of [1] to [10].

[12] A compound represented by the following General Formula (1).

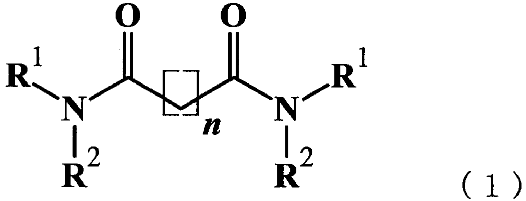

[In General Formula (1), R¹'s each independently represent POF₂ or SO₂F_{f},
R²'s each independently represent a hydrogen atom, a lithium ion, a sodium ion, a potassium ion, a linear alkyl group having 1 to 12 carbon atoms, or a branched alkyl group having 3 to 12 carbon atoms, and an oxygen atom may be included between a carbon atom-carbon atom bond in the alkyl group, any hydrogen atom of the alkyl group may be substituted with a fluorine atom, the alkyl group may contain an unsaturated bond, when R² represents a lithium ion, a sodium ion, or a potassium ion, a bond between a nitrogen atom and R² in General Formula (1) represents an ionic bond, and
n represents an integer of 0 to 3.]

[13] A compound represented by the following Formula (1a).

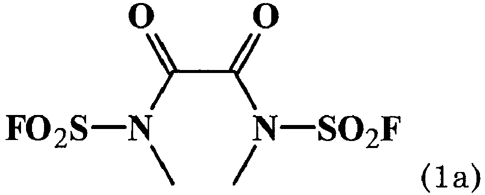

### ADVANTAGEOUS EFFECTS OF INVENTION

According to the present disclosure, a nonaqueous electrolytic solution and a nonaqueous electrolytic solution battery which have a low initial resistance value can be provided. A compound that can be suitably used in the above nonaqueous electrolytic solution can be provided.

### DESCRIPTION OF EMBODIMENTS

Configurations and combinations thereof in the following embodiments are merely examples, and various modifications are possible without departing from the scope of the present disclosure. In addition, the present disclosure is not limited by the embodiments, but only by the scope of claims.

Ranges expressed with "to" in the present specification mean ranges including numerical values indicated before and after "to" as a lower limit value and an upper limit value.

An initial resistance value in the present specification represents a resistance value of a nonaqueous electrolytic solution battery at a time of initial charge and discharge after an initial charge and discharge operation for battery stabilization. Specifically, the initial resistance value refers to a resistance value obtained by impedance measurement at the time of initial charge and discharge after three cycles of the initial charge and discharge operation for battery stabilization.

### [1. Nonaqueous Electrolytic Solution]

A nonaqueous electrolytic solution according to the present disclosure is a nonaqueous electrolytic solution containing a compound represented by the above General Formula (1), a solute, and a nonaqueous organic solvent.

### <(I) Regarding Compound Represented by General Formula (1)>

The nonaqueous electrolytic solution according to the present disclosure includes the compound represented by General Formula (1) (hereinafter, also referred to as "Component (I)" or simply "(I)").

When the nonaqueous electrolytic solution containing the above Component (I) is used in a nonaqueous electrolytic solution battery (for example, a lithium ion secondary battery or a sodium ion secondary battery), Component (I) decomposes on at least one of a positive electrode and a negative electrode, and forms a film having good ion conductivity on a surface of at least one of the positive electrode and the negative electrode. It is considered that this film prevents direct contact between the nonaqueous organic solvent or solute and an electrode active material, and reduces Li or Na ion dissociation energy of the solute. As a result, the present inventors presume that an effect of reducing an initial resistance of the nonaqueous electrolytic solution battery is achieved.

The compound represented by General Formula (1) is described below.

In General Formula (1), R¹'s each independently represent PO(R_{f})₂ or SO₂R_{f}.

R_{f}'s each independently represent a fluorine atom, a linear alkyl group having 1 to 4 carbon atoms, or a branched alkyl group having 3 to 4 carbon atoms, and an oxygen atom may be included between a carbon atom-carbon atom bond in the alkyl group, any hydrogen atom of the alkyl group may be substituted with a fluorine atom,

Specific examples of R_{f} representing a linear alkyl group having 1 to 4 carbon atoms or a branched alkyl group having 3 to 4 carbon atoms include a methyl group, an ethyl group, an n-propyl group, an isopropyl group, an n-butyl group, a sec-butyl group, an isobutyl group, a tert-butyl group, a trifluoromethyl group, a pentafluoroethyl group, a heptafluoropropyl group, a heptafluoroisopropyl group, and a nonafluoro-n-butyl group. Among the examples, the trifluoromethyl group is preferred.

R_{f} is preferably a fluorine atom.

Two R_{f}'s in PO(R_{f})₂ may be the same or different.

R¹'s are each independently preferably POF₂ or SO₂F, and both R¹'s are more preferably SO₂F.

In General Formula (1), R²'s each independently represent a hydrogen atom, a lithium ion, a sodium ion, a potassium ion, a linear alkyl group having 1 to 12 carbon atoms, or a branched alkyl group having 3 to 12 carbon atoms.

Specific examples of R² representing a linear alkyl group having 1 to 12 carbon atoms or a branched alkyl group having 3 to 12 carbon atoms include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the alkyl group. Specific examples of the above alkyl group containing an oxygen atom between a carbon atom-carbon atom bond include a 2-methoxyethyl group and a 2-ethoxyethyl group.

Any hydrogen atom of the above alkyl group may be substituted with a fluorine atom. Examples of the alkyl group in which any hydrogen atom is substituted with a fluorine atom include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

Further, the above alkyl group may contain an unsaturated bond. Examples of the alkyl group containing an unsaturated bond include a vinyl group, a 1-propenyl group, a 2-propenyl group, an ethynyl group, and a 2-propynyl group.

The above alkyl group is preferably an alkyl group having 6 or less carbon atoms because such an alkyl group can reduce resistance when a film is formed on an electrode. The above alkyl group is more preferably an alkyl group having 4 or less carbon atoms, and particularly preferably a methyl group, an ethyl group, an n-propyl group, an isopropyl group, an n-butyl group, or a tert-butyl group.

R²'s are each independently preferably a hydrogen atom, a lithium ion, a sodium ion, a linear alkyl group having 1 to 4 carbon atoms, or a branched alkyl group having 3 to 4 carbon atoms, more preferably a hydrogen atom, a lithium ion, a sodium ion, or a methyl group, and more preferably both R²'s are lithium ions or both R²'s are methyl groups.

n represents an integer of 0 to 3, preferably 0 or 1, and more preferably 0, from a viewpoint of keeping the resistance of the film as low as possible.

Specifically, the compound represented by General Formula (1) is preferably at least one selected from the group consisting of compounds represented by the following Formulas (1a) to (1z).

The compound represented by General Formula (1) is more preferably at least one selected from the group consisting of the compound represented by Formula (1a) (also referred to as Compound (1a)), the compound represented by Formula (1b) (also referred to as Compound (1b)), the compound represented by Formula (1c) (also referred to as Compound (1c)), the compound represented by Formula (1e) (also referred to as Compound (1e)), and the compound represented by Formula (1t) (also referred to as Compound (1t)), and particularly preferably is Compound (1a).

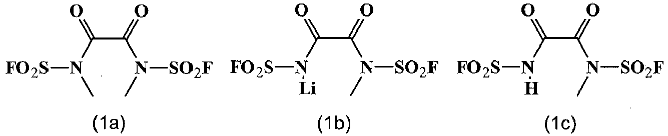

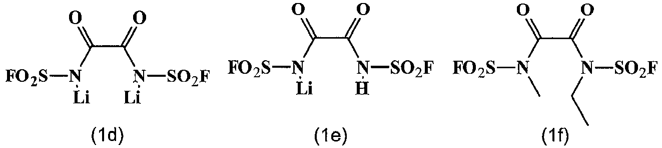

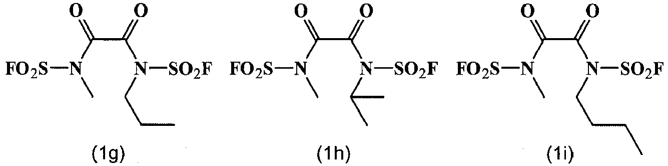

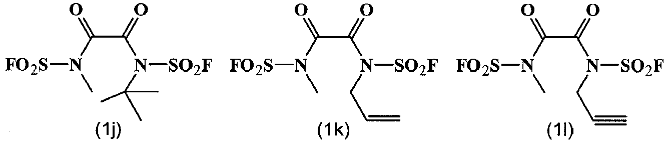

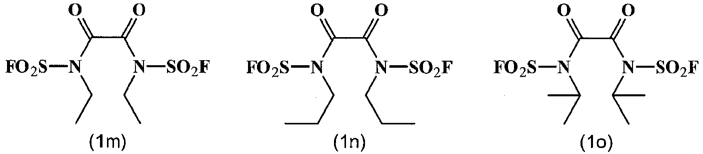

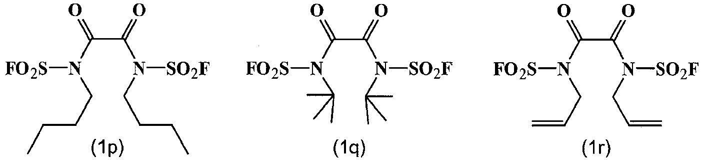

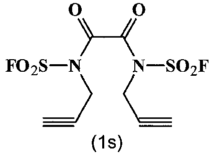

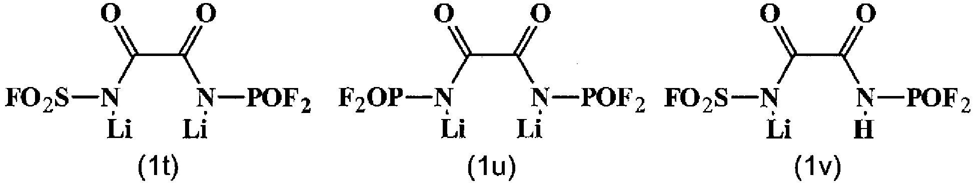

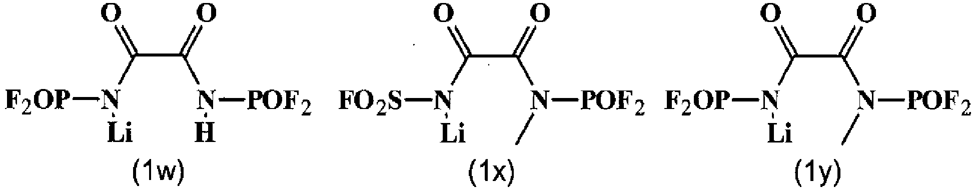

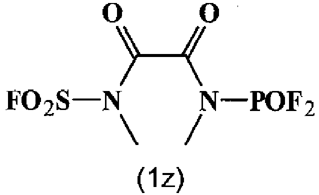

The present disclosure also relates to the compound represented by the above General Formula (1) and the above Compound (1a).

In the nonaqueous electrolytic solution according to the present disclosure, the compound represented by the above General Formula (1) is preferably used as an additive.

In the nonaqueous electrolytic solution according to the present disclosure, a lower limit of a total amount of the compound represented by the above General Formula (1) (hereafter, also described as "concentration of the compound represented by General Formula (1)") with respect to a total amount (100% by mass) of the compound represented by the above General Formula (1), the solute, and the nonaqueous organic solvent is preferably 0.001% by mass or more, more preferably 0.01% by mass or more, and still more preferably 0.1% by mass or more. An upper limit of the concentration of the compound represented by General Formula (1) is preferably 10.0% by mass or less, more preferably 5.0% by mass or less, and still more preferably 2.0% by mass or less.

By setting the concentration of the compound represented by General Formula (1) to 0.001% by mass or more, an effect of preventing an increase in the initial resistance of the nonaqueous electrolytic solution battery using the nonaqueous electrolytic solution is easily obtained. On the other hand, by setting the concentration of the compound represented by General Formula (1) to 10.0% by mass or less, an increase in viscosity of the nonaqueous electrolytic solution can be prevented, and an effect of improving high temperature cycle characteristics of the nonaqueous electrolytic solution battery using the nonaqueous electrolytic solution can be easily obtained.

In the nonaqueous electrolytic solution according to the present disclosure, as the compound represented by General Formula (1), one type of compound may be used alone, or two or more types of compounds may be mixed and used in any combination and ratio according to an application.

Although a method for synthesizing the compound represented by General Formula (1) is not particularly limited, for example, the following Compound (2a) can be synthesized by causing oxalyl chloride to react with MeSO₂NH₂ as described in Journal of Organic Chemistry (1964), 2592, (29).

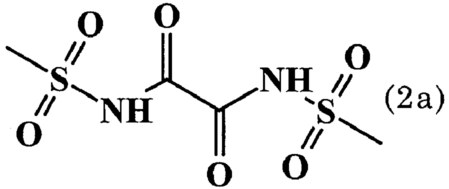

### <(II) Regarding Solute>

The nonaqueous electrolytic solution according to the present disclosure contains the solute.

Although the solute is not particularly limited, it is preferably an ionic salt, more preferably an ionic salt containing fluorine.

The solute is preferably, for example, an ionic salt containing a pair of at least one cation selected from the group consisting of alkali metal ions such as lithium ions and sodium ions, alkaline earth metal ions, and quaternary ammonium, and at least one anion selected from the group consisting of a hexafluorophosphate anion, a tetrafluoroborate anion, a perchlorate anion, a hexafluoroarsenate anion, a hexafluoroantimonate anion, a trifluoromethanesulfonate anion, a bis(trifluoromethanesulfonyl)imide anion, a bis(pentafluoroethanesulfonyl)imide anion, a (trifluoromethanesulfonyl)(pentafluoroethanesulfonyl)imide anion, a bis(fluorosulfonyl)imide anion, a (trifluoromethanesulfonyl)(fluorosulfonyl)imide anion, a (pentafluoroethanesulfonyl)(fluorosulfonyl)imide anion, a tris(trifluoromethanesulfonyl)methide anion, a bis(difluorophosphoryl)imide anion, a (difluorophosphoryl)(trifluoromethanesulfonyl)imide anion, a (difluorophosphoryl)(fluorosulfonyl)imide anion, and a difluorophosphate anion.

One type of these solutes may be used alone, or two or more types may be mixed and used in any combination and any ratio according to an application.

Among the above solutes, considering energy density, output characteristics, life, and the like of a nonaqueous electrolytic solution battery, the cation is preferably at least one selected from the group consisting of lithium, sodium, magnesium, and quaternary ammonium, and the anion is preferably at least one selected from the group consisting of a hexafluorophosphate anion, a tetrafluoroborate anion, a bis(trifluoromethanesulfonyl)imide anion, a bis(fluorosulfonyl)imide anion, a bis(difluorophosphoryl)imide anion, a (difluorophosphoryl)(fluorosulfonyl)imide anion, and a difluorophosphate anion.

A total amount of the solute in the nonaqueous electrolytic solution according to the present disclosure (hereinafter, also described as "solute concentration") is not particularly limited, but a lower limit is preferably 0.5 mol/L or more, more preferably 0.7 mol/L or more, and still more preferably 0.9 mol/L or more. In addition, an upper limit of the solute concentration is preferably 5.0 mol/L or less, more preferably 4.0 mol/L or less, and still more preferably 2.0 mol/L or less. By setting the solute concentration to 0.5 mol/L or more, it is possible to prevent deterioration of the cycle characteristics and the output characteristics of the nonaqueous electrolytic solution battery due to deterioration in ionic conductivity, and by setting the solute concentration to 5.0 mol/L or less, it is possible to prevent the deterioration in ionic conductivity, cycle characteristics, and output characteristics of the nonaqueous electrolytic solution battery due to an increase in the viscosity of the nonaqueous electrolytic solution.

### <(III) Regarding Nonaqueous Organic Solvent>

The type of the nonaqueous organic solvent used in the nonaqueous electrolytic solution according to the present disclosure is not particularly limited as long as the solvent can dissolve the above (I) and (II), and for example, any nonaqueous organic solvent such as carbonates, esters, ethers, lactones, nitriles, imides, and sulfones can be used.

Specifically, the nonaqueous organic solvent is preferably at least one selected from the group consisting of ethyl methyl carbonate (hereinafter, also described as "EMC"), dimethyl carbonate (hereinafter, also described as "DMC"), diethyl carbonate (hereinafter, also described as "DEC"), methyl propyl carbonate, ethyl propyl carbonate, methyl butyl carbonate, 2,2,2-trifluoroethyl methyl carbonate, 2,2,2-trifluoroethyl ethyl carbonate, 2,2,2-trifluoroethyl propyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter also described as "EC"), propylene carbonate (hereinafter, also described as "PC"), butylene carbonate, fluoroethylene carbonate (hereinafter, also described as "FEC"), difluoroethylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethylsulfoxide, sulfolane, γ-butyrolactone, and γ-valerolactone.

In the present disclosure, an ionic liquid having a salt structure may be used as the nonaqueous organic solvent.

It is preferable that the above nonaqueous organic solvent is at least one selected from the group consisting of cyclic carbonates and chain carbonates from a viewpoint of excellent cycle characteristics at high temperatures. In addition, it is preferable that the above nonaqueous organic solvent is at least one selected from the group consisting of esters from a viewpoint of excellent input and output characteristics at low temperatures.

Specific examples of the above cyclic carbonate include EC, PC, butylene carbonate, and FEC, and at least one selected from the group consisting of EC, PC, and FEC is preferable.

Specific examples of the above chain carbonate include EMC, DMC, DEC, methyl propyl carbonate, ethyl propyl carbonate, 2,2,2-trifluoroethyl methyl carbonate, 2,2,2-trifluoroethyl ethyl carbonate, 1,1,1,3,3,3-hexafluoro-1-propylmethyl carbonate, and 1,1,1,3,3,3-hexafluoro-1-propylethyl carbonate, and at least one selected from the group consisting of EMC, DMC, DEC, and methyl propyl carbonate is preferable.

Specific examples of the above ester include methyl acetate, ethyl acetate, methyl propionate, ethyl propionate, methyl 2-fluoropropionate, and ethyl 2-fluoropropionate.

### <Regarding Another Additive>

An additive component commonly used in the nonaqueous electrolytic solution according to the present disclosure may be further added in any ratio as long as the gist of the present disclosure is not impaired.

Specific examples of another additive include compounds that have an overcharge prevention effect, a negative electrode film-forming effect, and a positive electrode protective effect, such as cyclohexylbenzene, cyclohexylfluorobenzene, fluorobenzene, biphenyl, difluoroanisole, tert-butylbenzene, tert-amylbenzene, 2-fluorotoluene, 2-fluorobiphenyl, vinylene carbonate, dimethylvinylene carbonate, vinylethylene carbonate, fluoroethylene carbonate, trans-difluoroethylene carbonate, methyl propargyl carbonate, ethyl propargyl carbonate, dipropargyl carbonate, maleic anhydride, succinic anhydride, methylene methanedisulfonate, dimethylene methanedisulfonate, trimethylene methanedisulfonate, methyl methanesulfonate, 1,6-diisocyanatohexane, tris(trimethylsilyl)borate, succinonitrile, (ethoxy)pentafluorocyclotriphosphazene, lithium difluorobis(oxalato)phosphate, sodium difluorobis(oxalato)phosphate, potassium difluorobis(oxalato)phosphate, lithium difluorooxalatoborate, sodium difluorooxalatoborate, potassium difluorooxalatoborate, lithium bis(oxalato)borate, sodium bis(oxalato)borate, potassium bis(oxalato)borate, lithium tetrafluorooxalatophosphate, sodium tetrafluorooxalatophosphate, potassium tetrafluorooxalatophosphate, lithium tris(oxalato)phosphate, lithium ethylfluorophosphate, lithium fluorophosphate, ethenesulfonyl fluoride, lithium fluorosulfonate, trifluoromethanesulfonyl fluoride, methanesulfonyl fluoride, and phenyl difluorophosphate.

The nonaqueous electrolytic solution according to the present disclosure may further contain at least one compound selected from the group consisting of compounds represented by the following General Formulas (2) to (3).

A content of at least one compound selected from the group consisting of compounds represented by the following General Formulas (2) to (3) is preferably 0.01% by mass to 5.0% by mass with respect to the total amount of the nonaqueous electrolytic solution.

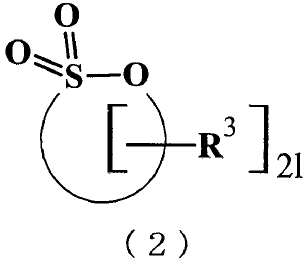

[General formula (2) is a compound having a cyclic skeleton including 4 to 7 atoms selected from any of carbon atoms, oxygen atoms, and sulfur atoms, and has at least one adjacent sulfonyl group and oxygen atom in the cyclic skeleton. The cyclic skeleton may contain an unsaturated bond. A state of the sulfur atom forming the cyclic skeleton is selected from any of S, SO, and SO₂. l represents the number of carbon atoms contained in the cyclic skeleton. R³'s are bonded to carbon atoms forming the cyclic skeleton, and each independently represent a hydrogen atom, a fluorine atom, or an aliphatic hydrocarbon group having 1 to 6 carbon atoms, and the aliphatic hydrocarbon group may contain at least one of an oxygen atom and an unsaturated bond, and any hydrogen atom of the aliphatic hydrocarbon group may be substituted with a fluorine atom. When the carbon atoms forming the cyclic skeleton are adjacent to each other, these carbon atoms may be combined to form an aromatic ring, and a hydrocarbon group having 1 to 4 carbon atoms is bonded to the aromatic ring. The hydrocarbon group bonded to the aromatic ring may contain at least one of an oxygen atom and an unsaturated bond, and any hydrogen atom of the hydrocarbon group may be substituted with a fluorine atom.]

The cyclic skeleton of General Formula (2) has 4 to 7 atoms selected from any of carbon atoms, oxygen atoms or sulfur atoms as ring members. The above cyclic skeleton includes one S (sulfur atom) as the ring member and one O (oxygen atom) bonded to the sulfur atom by a single bond in a -S(=O)₂-O- site described in General Formula (2), and has 4 to 7 atoms selected from any of carbon atoms, oxygen atoms, and sulfur atoms as the ring members.

The compound having the above cyclic skeleton is preferable in that other than the -S(=O)₂-O-site, -CH₂-CH₂-CH₂-, -CH₂-CH₂-CH₂-CH₂-, -CH₂-CH=CH-, -CH₂-CH₂-O-, -CH₂-CH₂-CH₂-O-, -CH₂-S(=O)₂-O-CH₂-, -S(=O)₂-CH₂-CH₂-, and -S(=O)₂-CH₂-CH₂-CH₂- are connected to the oxygen atom in order and a film having high durability in electrode can be formed.

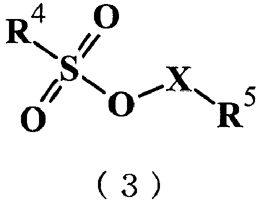

[In General Formula (3), X represents a sulfonyl group or a ketone group, and R⁴ and R⁵ are each independently selected from aliphatic hydrocarbon groups having 1 to 10 carbon atoms. At least one selected from the group consisting of a fluorine atom, an oxygen atom, an unsaturated bond, and an ester bond may be present in the aliphatic hydrocarbon group.]

The nonaqueous electrolytic solution according to the present disclosure may contain a compound represented by the following General Formula (4).

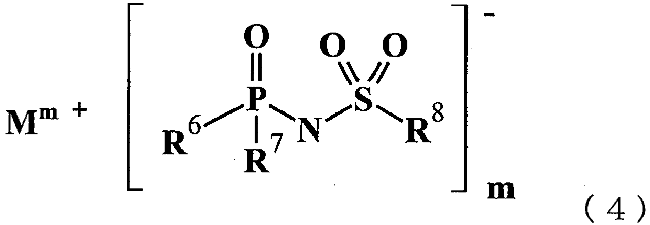

[In General Formula (4), R⁶ to R⁸ are each independently an organic group selected from a fluorine atom, a linear alkyl group having 1 to 10 carbon atoms, a branched alkyl group having 3 to 10 carbon atoms, a linear alkoxy group having 1 to 10 carbon atoms, a branched alkoxy group having 3 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 10 carbon atoms, a cycloalkoxy group having 3 to 10 carbon atoms, a cycloalkenyl group having 3 to 10 carbon atoms, a cycloalkenyloxy group having 3 to 10 carbon atoms, an aryl group having 6 to 10 carbon atoms, and an aryloxy group having 6 to 10 carbon atoms, and the organic group may have a fluorine atom, an oxygen atom, or an unsaturated bond. Here, at least one of R⁶ to R⁸ is a fluorine atom.

M^{m+} is an alkali metal cation, an alkaline earth metal cation, or an onium cation, and m represents the same integer as a valence of the corresponding cation.]

When the compound (salt having an imide anion) represented by General Formula (4) has at least one P-F bond or S-F bond, excellent low temperature characteristics can be obtained. The larger the number of P-F bonds and S-F bonds in the salt having the above imide anion, the more the low temperature characteristics can be further improved, which is preferable, and in the salt having the imide anion represented by the above General Formula (4), a compound in which all of R⁶ to R⁸ are fluorine atoms is more preferable.

In the salt having the imide anion represented by the above General Formula (4), it is preferable that
at least one of R⁶ to R⁸ is a fluorine atom, and
at least one of R⁶ to R⁸ is a compound which may contain fluorine atoms and is selected from hydrocarbon groups having 6 or less carbon atoms.

In the salt having the imide anion represented by the above General Formula (4), it is preferable that
at least one of R⁶ to R⁸ is a fluorine atom, and
at least one of R⁶ to R⁸ is a compound selected from a methyl group, a methoxy group, an ethyl group, an ethoxy group, a propyl group, a propoxyl group, a vinyl group, an allyl group, an allyloxy group, an ethynyl group, a 2-propynyl group, a 2-propynyloxy group, a phenyl group, phenyloxy group, a 2,2-difluoroethyl group, a 2,2-difluoroethyloxy group, a 2,2,2-trifluoroethyl group, a 2,2,2-trifluoroethyloxy group, a 2,2,3,3-tetrafluoropropyl group, a 2,2,3,3-tetrafluoropropyloxy group, a 1,1,1,3,3,3-hexafluoroisopropyl group, and a 1,1,1,3,3,3-hexafluoroisopropyloxy group.

A counter cation M^{m+} of the salt having the imide anion represented by General Formula (4) is preferably selected from the group consisting of lithium ions, sodium ions, potassium ions, and tetraalkylammonium ions.

In the above General Formula (4), examples of the alkyl group and the alkoxy group represented by R⁶ to R⁸ include alkyl groups having 1 to 10 carbon atoms, such as a methyl group, an ethyl group, a propyl group, an isopropyl group, a butyl group, a sec-butyl group, a tert-butyl group, a pentyl group, a 2,2-difluoroethyl group, 2,2,2-trifluoroethyl group, 2,2,3,3-tetrafluoropropyl group, and a 1,1,1,3,3,3-hexafluoroisopropyl group, fluorine-containing alkyl groups, and alkoxy groups derived from these groups.

Examples of the alkenyl group and the alkenyloxy group include alkenyl groups having 2 to 10 carbon atoms such as a vinyl group, an allyl group, a 1-propenyl group, an isopropenyl group, a 2-butenyl group, and a 1,3-butadienyl group, fluorine-containing alkenyl groups, and alkenyloxy groups derived from these groups.

Examples of the alkynyl group and the alkynyloxy group include alkynyl groups having 2 to 10 carbon atoms such as an ethynyl group, a 2-propynyl group, and a 1,1-dimethyl-2-propynyl group, fluorine-containing alkynyl groups, and alkynyloxy groups derived from these groups.

Examples of the cycloalkyl group and the cycloalkoxy group include cycloalkyl groups having 3 to 10 carbon atoms such as a cyclopentyl group and a cyclohexyl group, fluorine-containing cycloalkyl groups, and cycloalkoxy groups derived from these groups.

Examples of the cycloalkenyl group and the cycloalkenyloxy group include cycloalkenyl groups having 3 to 10 carbon atoms such as a cyclopentenyl group and a cyclohexenyl group, fluorine-containing cycloalkenyl groups, and cycloalkenyloxy groups derived from these groups.

Examples of the aryl group and the aryloxy group include aryl groups having 6 to 10 carbon atoms such as a phenyl group, a tolyl group, and an xylyl group, fluorine-containing aryl groups, and aryloxy groups derived from these groups.

Specific examples and synthesis methods of the salt having the imide anion represented by General Formula (4) include those described in WO2017/111143.

A content of another additive in the nonaqueous electrolytic solution is preferably 0.01% by mass or more and 8.0% by mass or less with respect to the total amount of the nonaqueous electrolytic solution.

When the ionic salt exemplified as the solute is less than 0.5 mol/L, which is the lower limit of the suitable concentration of the solute, in the nonaqueous electrolytic solution, the ionic salt can exert a negative electrode film-forming effect and a positive electrode protective effect as "another additive". In this case, the content in the nonaqueous electrolytic solution is preferably 0.01% by mass to 5.0% by mass.

Examples of the ionic salt in this case include lithium trifluoromethanesulfonate, sodium trifluoromethanesulfonate, potassium trifluoromethanesulfonate, magnesium trifluoromethanesulfonate, lithium bis(trifluoromethanesulfonyl)imide, sodium bis(trifluoromethanesulfonyl)imide, potassium bis(trifluoromethanesulfonyl)imide, magnesium bis(trifluoromethanesulfonyl)imide, lithium bis(fluorosulfonyl)imide, sodium bis(fluorosulfonyl)imide, potassium bis(fluorosulfonyl)imide, magnesium bis(fluorosulfonyl)imide, lithium (trifluoromethanesulfonyl)(fluorosulfonyl)imide, sodium (trifluoromethanesulfonyl)(fluorosulfonyl)imide, potassium (trifluoromethanesulfonyl)(fluorosulfonyl)imide, magnesium (trifluoromethanesulfonyl)(fluorosulfonyl)imide, lithium bis(difluorophosphoryl)imide, sodium bis(difluorophosphoryl)imide, potassium bis(difluorophosphoryl)imide, magnesium bis(difluorophosphoryl)imide, lithium (difluorophosphoryl)(fluorosulfonyl)imide, sodium (difluorophosphoryl)(fluorosulfonyl)imide, potassium (difluorophosphoryl)(fluorosulfonyl)imide, magnesium (difluorophosphoryl)(fluorosulfonyl)imide, lithium (difluorophosphoryl)(trifluoromethanesulfonyl)imide, sodium (difluorophosphoryl)(trifluoromethanesulfonyl)imide, potassium (difluorophosphoryl)(trifluoromethanesulfonyl)imide, magnesium (difluorophosphoryl)(trifluoromethanesulfonyl)imide, lithium difluorophosphate, and sodium difluorophosphate.

In addition, alkali metal salts other than the above solutes (lithium salt, sodium salt, potassium salt, magnesium salt) may be used as additives.

Specific examples include carboxylates such as lithium acrylate, sodium acrylate, lithium methacrylate, and sodium methacrylate, and sulfate ester salts such as lithium methyl sulfate, sodium methyl sulfate, lithium ethyl sulfate, and sodium ethyl sulfate.

The nonaqueous electrolytic solution according to the present disclosure is preferable to contain, with respect to the total amount of the nonaqueous electrolytic solution, 0.01% by mass to 5.0% by mass of at least one selected from vinylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, and lithium (difluorophosphoryl)(fluorosulfonyl)imide in the above another additive.

At least one selected from lithium difluorooxalatoborate, lithium (difluorophosphoryl)(fluorosulfonyl)imide, and lithium difluorobis(oxalato)phosphate is more preferable from a viewpoint of preventing an increase in the initial resistance value.

In addition, the nonaqueous electrolytic solution according to the present disclosure can also contain a polymer, and as in the case of being used in a nonaqueous electrolytic solution battery referred to as a polymer battery, the nonaqueous electrolytic solution can be quasi-solidified with a gelling agent or a cross-linked polymer. A polymer solid electrolyte includes one containing the nonaqueous organic solvent as a plasticizer.

The above polymer is not particularly limited as long as the polymer is an aprotic polymer capable of dissolving the compound represented by the above General Formula (1), the above solute, and the above another additive. Examples of the polymer include polymers having polyethylene oxide as a main chain or a side chain, homopolymers or copolymers of polyvinylidene fluoride, methacrylic acid ester polymers, and polyacrylonitrile. When a plasticizer is added to these polymers, an aprotic nonaqueous organic solvent is preferred among the above nonaqueous organic solvents.

### [2. Nonaqueous Electrolytic Solution Battery]

The nonaqueous electrolytic solution battery according to the present disclosure at least includes the nonaqueous electrolytic solution according to the present disclosure described above, a negative electrode, and a positive electrode. Furthermore, a separator, an exterior body, and the like are preferably included.

Although the negative electrode is not particularly limited, it is preferable to use a material capable of reversibly intercalating and deintercalating alkali metal ions such as lithium ions and sodium ions, or alkaline earth metal ions.

Although the positive electrode is not particularly limited, it is preferable to use a material capable of reversibly intercalating and deintercalating alkali metal ions such as lithium ions and sodium ions, or alkaline earth metal ions.

For example, when the cation is lithium, lithium metal, alloys and intermetallic compounds of lithium with other metals, various carbon materials capable of absorbing and desorbing lithium, metal oxides, metal nitrides, activated carbon, conductive polymers, and the like are used as negative electrode materials. Examples of the above carbon material include graphitizable carbon, non-graphitizable carbon (also referred to as hard carbon) having a (002) plane spacing of 0.37 nm or more, and graphite having a (002) plane spacing of 0.37 nm or less, and for the graphite, artificial graphite, natural graphite, and the like are used.

For example, when the cation is lithium, lithium-containing transition metal composite oxides such as LiCoO₂, LiNiO₂, LiMnO₂, LiMn₂O₄, a mixture of a plurality of transition metals such as Co, Mn, and Ni and these lithium-containing transition metal composite oxides, those in which a part of the transition metals of these lithium-containing transition metal composite oxides is replaced by a metal other than the transition metals, phosphate compounds of transition metals such as LiFePO₄, LiCoPO₄, LiMnPO₄ referred to as olivine, oxides such as TiO₂, V₂O₅, and MoOs, sulfides such as TiS₂ and FeS, conductive polymers such as polyacetylene, polyparaphenylene, polyaniline, and polypyrrole, activated carbon, radical-generating polymers, carbon materials, and the like, can be used as a positive electrode material.

As the positive electrode and negative electrode materials, acetylene black, ketjen black, carbon fiber, or graphite as a conductive material, and polytetrafluoroethylene, polyvinylidene fluoride, SBR resin, or the like as a binder are added, and an electrode sheet molded into a sheet shape can be used.

As a separator for preventing contact between the positive electrode and the negative electrode, a nonwoven fabric or porous sheet made of polyolefin, polyethylene, paper, glass fiber, or the like is used.

An electrochemical device having a shape such as a coin shape, a cylindrical shape, a square shape, or an aluminum laminate sheet shape is assembled based on the above elements.

### [Examples]

The present disclosure will be described in more detail below with reference to Examples, but the present disclosure is not limited by these descriptions.

### <Synthesis Example 1: Synthesis of Compound (1a)>

1.0 g of oxalyl chloride, 10.0 g of ethyl methyl carbonate, and 7.80 g of N-methylsulfamoyl fluoride were added in a 100 ml two-necked flask, and 0.96 g of N,N,N',N'-tetramethylethylenediamine was added dropwise while stirring at 0°C. After confirming production of an object product by NMR, 20 g of diethyl ether was added, and then washed with water twice. An oil layer was dried with magnesium sulfate and then concentrated to dryness to obtain 0.6 g of a white solid (yield: 27%). Compound (1a):
¹⁹F-NMR [reference substance; CFCl₃, heavy solvent CD₃CN], δppm; 50.26 (s, 2F)
¹H-NMR [reference substance; (CH₃)₄Si, heavy solvent CD₃CN], δppm; 3.401 (s, 6H)

### [Preparation of Nonaqueous Electrolytic Solutions of Examples and Comparative Examples]

### <Comparative Example 1-1>

### (Preparation of LiPF₆ Solution)

EC, FEC, EMC, and DMC were mixed in a volume ratio of EC:FEC:EMC:DMC = 2:1:3:4 in a glove box with a dew point of -60°C or lower. After that, LiPF₆ with an amount which is a concentration of 1.0 mol/L was added while maintaining an internal temperature at 40°C or lower, was stirred, and completely dissolved to obtain a LiPF₆ solution. This LiPF₆ solution was set as Comparative Nonaqueous Electrolytic Solution 1-1.

### <Example 1-1>

### (Preparation of Nonaqueous Electrolytic Solution 1-1)

EC, FEC, EMC, and DMC were mixed in a volume ratio of EC:FEC:EMC:DMC = 2:1:3:4 in a glove box with a dew point of -60°C or lower. After that, LiPF₆ of the 1.0 mol/L was added while maintaining the internal temperature 40°C or lower, Compound (1a) corresponding to the compound represented by General Formula (1) was added to 0.5% by mass with respect to a total amount of the nonaqueous organic solvent, the solute, and Compound (1a), was stirred and dissolved for one hour to obtain Nonaqueous Electrolytic Solution 1-1 according to Example 1-1.

### <Example 1-2, Comparative Examples 1-2 to 1-5>

### (Preparation of Nonaqueous Electrolytic Solution 1-2 and Comparative Nonaqueous Electrolytic solutions 1-2 to 1-5)

Except that the type and adding amounts of the compound represented by General Formula (1) (and comparative compound) are changed as described in Table 1, Nonaqueous Electrolytic Solution 1-2 and Comparative Nonaqueous Electrolytic Solutions 1-2 to 1-5 were obtained in the same way as the preparation of Nonaqueous Electrolytic Solution 1-1. In the subsequent table, VC means vinylene carbonate, BOB means lithium bis(oxalato)borate, DFOB means lithium difluorooxalatoborate, TFOP means lithium tetrafluorooxalatophosphate, DFBOP means lithium difluorobisoxalatephosphate, FS means lithium fluorosulfonate, FSI means lithium bis(fluorosulfonyl)imide, DFP means lithium difluorophosphate, and PRS means 1,3-propenesultone.

**[Table 1]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 1-1 | Nonaqueous Electrolytic Solution 1-1 | (1a) | 0.5 | - | - | 80.3 |
| Example 1-2 | Nonaqueous Electrolytic Solution 1-2 | (1a) | 1.0 | - | - | 73.5 |
| Comparative Example 1-1 | Comparative Nonaqueous Electrolytic Solution 1-1 | - | - | - | - | 100 |
| Comparative Example 1-2 | Comparative Nonaqueous Electrolytic Solution 1-2 | DFP | 0.5 | - | - | 87.6 |
| Comparative Example 1-3 | Comparative Nonaqueous Electrolytic Solution 1-3 | DFP | 1.0 | - | | 85.3 |
| Comparative Example 1-4 | Comparative Nonaqueous Electrolytic Solution 1-4 | FS | 0.5 | - | | 90.8 |
| Comparative Example 1-5 | Comparative Nonaqueous Electrolytic Solution 1-5 | FS | 1.0 | - | | 87.7 |

### <Example 2-1, Comparative Examples 2-1 to 2-3>

### (Preparation of Nonaqueous Electrolytic Solution 2-1 and Comparative Nonaqueous Electrolytic Solutions 2-1 to 2-3)

Furthermore, except that vinylene carbonate as another additive was added to a concentration described in Table 2 and dissolved, Nonaqueous Electrolytic Solution 2-1 and Comparative Nonaqueous Electrolytic Solutions 2-1 to 2-3 were respectively obtained in the same way as the preparation of Nonaqueous Electrolytic Solution 1-2 and Comparative Nonaqueous Electrolytic Solutions 1-1, 1-3, and 1-5.

**[Table 2]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 2-1 | Nonaqueous Electrolytic Solution 2-1 | (1a) | 1.0 | VC | 1.0 | 74.1 |
| Comparative Example 2-1 | Comparative Nonaqueous Electrolytic Solution 2-1 | - | - | VC | 1.0 | 100 |
| Comparative Example 2-2 | Comparative Nonaqueous Electrolytic Solution 2-2 | DFP | 1.0 | VC | 1.0 | 88.2 |
| Comparative Example 2-3 | Comparative Nonaqueous Electrolytic Solution 2-3 | FS | 1.0 | VC | 1.0 | 90.7 |

### <Example 3-1, Comparative Examples 3-1 to 3-3>

### (Preparation of Nonaqueous Electrolytic Solution 3-1 and Comparative Nonaqueous Electrolytic Solutions 3-1 to 3-3)

Except that vinylene carbonate was changed to lithium bis(oxalato)borate, Nonaqueous Electrolytic Solution 3-1 and Comparative Nonaqueous Electrolytic Solutions 3-1 to 3-3 were respectively obtained in the same way as the preparation of Nonaqueous Electrolytic Solution 2-1 and Comparative Nonaqueous Electrolytic Solutions 2-1 to 2-3.

**[Table 3]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 3-1 | Nonaqueous Electrolytic Solution 3-1 | (1a) | 1.0 | BOB | 1.0 | 80.1 |
| Comparative Example 3-1 | Comparative Nonaqueous Electrolytic Solution 3-1 | - | - | BOB | 1.0 | 100 |
| Comparative Example 3-2 | Comparative Nonaqueous Electrolytic Solution 3-2 | DFP | 1.0 | BOB | 1.0 | 88.8 |
| Comparative Example 3-3 | Comparative Nonaqueous Electrolytic Solution 3-3 | FS | 1.0 | BOB | 1.0 | 91.2 |

### <Example 4-1, Comparative Examples 4-1 to 4-3>

### (Preparation of Nonaqueous Electrolytic Solution 4-1 and Comparative Nonaqueous Electrolytic Solutions 4-1 to 4-3)

Except that vinylene carbonate was changed to lithium difluorooxalatoborate, Nonaqueous Electrolytic Solution 4-1 and Comparative Nonaqueous Electrolytic Solutions 4-1 to 4-3 were respectively obtained in the same way as the preparation of Nonaqueous Electrolytic Solution 2-1 and Comparative Nonaqueous Electrolytic Solutions 2-1 to 2-3.

**[Table 4]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 4-1 | Nonaqueous Electrolytic Solution 4-1 | (1a) | 1.0 | DFOB | 1.0 | 72.1 |
| Comparative Example 4-1 | Comparative Nonaqueous Electrolytic Solution 4-1 | - | - | DFOB | 1.0 | 100 |
| Comparative Example 4-2 | Comparative Nonaqueous Electrolytic Solution 4-2 | DFP | 1.0 | DFOB | 1.0 | 74.7 |
| Comparative Example 4-3 | Comparative Nonaqueous Electrolytic Solution 4-3 | FS | 1.0 | DFOB | 1.0 | 76.8 |

### <Example 5-1, Comparative Examples 5-1 to 5-3>

### (Preparation of Nonaqueous Electrolytic Solution 5-1 and Comparative Nonaqueous Electrolytic Solutions 5-1 to 5-3)

Except that vinylene carbonate was changed to lithium difluorobis(oxalato)phosphate, Nonaqueous Electrolytic Solution 5-1 and Comparative Nonaqueous Electrolytic Solutions 5-1 to 5-3 were respectively obtained in the same way as the preparation of Nonaqueous Electrolytic Solution 2-1 and Comparative Nonaqueous Electrolytic Solutions 2-1 to 2-3.

**[Table 5]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 5-1 | Nonaqueous Electrolytic Solution 5-1 | (1a) | 1.0 | DFBOP | 1.0 | 76.8 |
| Comparative Example 5-1 | Comparative Nonaqueous Electrolytic Solution 5-1 | - | - | DFBOP | 1.0 | 100 |
| Comparative Example 5-2 | Comparative Nonaqueous Electrolytic Solution 5-2 | DFP | 1.0 | DFBOP | 1.0 | 87.9 |
| Comparative Example 5-3 | Comparative Nonaqueous Electrolytic Solution 5-3 | FS | 1.0 | DFBOP | 1.0 | 90.4 |

### <Example 6-1, Comparative Examples 6-1 to 6-3>

### (Preparation of Nonaqueous Electrolytic Solution 6-1 and Comparative Nonaqueous Electrolytic Solutions 6-1 to 6-3)

Except that vinylene carbonate was changed to lithium tetrafluorooxalatophosphate, Nonaqueous Electrolytic Solution 6-1 and Comparative Nonaqueous Electrolytic Solutions 6-1 to 6-3 were respectively obtained in the same way as the preparation of Nonaqueous Electrolytic Solution 2-1 and Comparative Nonaqueous Electrolytic Solutions 2-1 to 2-3.

**[Table 6]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 6-1 | Nonaqueous Electrolytic Solution 6-1 | (1a) | 1.0 | TFOP | 1.0 | 76.2 |
| Comparative Example 6-1 | Comparative Nonaqueous Electrolytic Solution 6-1 | - | - | TFOP | 1.0 | 100 |
| Comparative Example 6-2 | Comparative Nonaqueous Electrolytic Solution 6-2 | DFP | 1.0 | TFOP | 1.0 | 87.0 |
| Comparative Example 6-3 | Comparative Nonaqueous Electrolytic Solution 6-3 | FS | 1.0 | TFOP | 1.0 | 90.1 |

### <Example 7-1, Comparative Examples 7-1 to 7-3>

### (Preparation of Nonaqueous Electrolytic Solution 7-1 and Comparative Nonaqueous Electrolytic Solutions 7-1 to 7-3)

Except that vinylene carbonate was changed to lithium bis(fluorosulfonyl)imide, Nonaqueous Electrolytic Solution 7-1 and Comparative Nonaqueous Electrolytic Solutions 7-1 to 7-3 were respectively obtained in the same way as the preparation of Nonaqueous Electrolytic Solution 2-1 and Comparative Nonaqueous Electrolytic Solutions 2-1 to 2-3.

**[Table 7]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 7-1 | Nonaqueous Electrolytic Solution 7-1 | (1a) | 1.0 | FSI | 1.0 | 76.5 |
| Comparative Example 7-1 | Comparative Nonaqueous Electrolytic Solution 7-1 | - | - | FSI | 1.0 | 100 |
| Comparative Example 7-2 | Comparative Nonaqueous Electrolytic Solution 7-2 | DFP | 1.0 | FSI | 1.0 | 85.2 |
| Comparative Example 7-3 | Comparative Nonaqueous Electrolytic Solution 7-3 | FS | 1.0 | FSI | 1.0 | 87.7 |

### <Example 8-1, Comparative Examples 8-1 to 8-3>

### (Preparation of Nonaqueous Electrolytic Solution 8-1 and Comparative Nonaqueous Electrolytic Solutions 8-1 to 8-3)

Except that vinylene carbonate was changed to 1,3-propenesultone, Nonaqueous Electrolytic Solution 8-1 and Comparative Nonaqueous Electrolytic Solutions 8-1 to 8-3 were respectively obtained in the same way as the preparation of Nonaqueous Electrolytic Solution 2-1 and Comparative Nonaqueous Electrolytic Solutions 2-1 to 2-3.

**[Table 8]**

| | Nonaqueous Electrolytic Solution | Compound represented by General Formula (1) (comparative compound) | | Another additive | | Initial resistance (relative value) |
|---|---|---|---|---|---|---|
| | | Type | Content [% by mass] | Type | Content [% by mass] | |
| Example 8-1 | Nonaqueous Electrolytic Solution 8-1 | (1a) | 1.0 | PRS | 1.0 | 73.6 |
| Comparative Example 8-1 | Comparative Nonaqueous Electrolytic Solution 8-1 | - | - | PRS | 1.0 | 100 |
| Comparative Example 8-2 | Comparative Nonaqueous Electrolytic Solution 8-2 | DFP | 1.0 | PRS | 1.0 | 86.8 |
| Comparative Example 8-3 | Comparative Nonaqueous Electrolytic Solution 8-3 | FS | 1.0 | PRS | 1.0 | 87.8 |

In the above Tables 1 to 8, the adding amount of the compound represented by General Formula (1) (and comparative compounds DFP and FS) indicates a concentration with respect to the total amount of the nonaqueous solvent, the solute, and the compound. Further, the adding amount of another additive indicates a concentration with respect to the total amount of the nonaqueous solvents, solutes, compounds, and another additive.

### [Production of Nonaqueous Electrolytic Solution Battery]

### (Production of NCM622 Positive Electrode)

3.5% by mass of polyvinylidene fluoride (hereinafter, also described as PVDF) as a binder and 4.5% by mass of acetylene black as a conductive material were mixed to 92% by mass of LiNi_{0.6}Co_{0.2}Mn_{0.2}O₂ powder, and N-methyl-2-pyrrolidone was further added to produce a positive electrode mixture paste. The paste was applied onto both sides of aluminum foil (A1085), dried and pressed, and then punched into 4 cm × 5 cm to obtain an NCM622 positive electrode for test.

### <Production of Artificial Graphite Negative Electrode>

2% by mass of styrene-butadiene rubber, 1% by mass of sodium carboxymethylcellulose, and water were mixed to 97% by mass of artificial graphite powder to produce a negative electrode mixture paste. The paste was applied onto one side of copper foil, dried and pressed, and then punched into 4 cm × 5 cm to obtain an artificial graphite negative electrode for test.

### (Production of Nonaqueous Electrolytic Solution Battery)

Terminals were welded to the above NCM622 positive electrode under an argon atmosphere at a dew point of -50°C or lower, and both sides thereof were then stacked between two polyethylene separators (5 cm × 6 cm), and outsides thereof were stacked between two artificial graphite negative electrodes to which terminals had been welded in advance so that a surface of the negative electrode active material faces opposite to a surface of the positive electrode active material. The stacked product was put in an aluminum laminated bag having an opening on one side, the nonaqueous electrolytic solution was vacuum-injected into the bag, and the opening was then sealed with heat to produce aluminum laminated nonaqueous electrolytic solution batteries according to Examples and Comparative Examples. The nonaqueous electrolytic solution used those described in Tables 1 to 8.

### [Evaluation]

### -Initial Charge and Discharge-

A nonaqueous electrolytic solution battery was put in a 25°C constant temperature bath and, in this state, connected to a charge and discharge device. Charge was performed at 3 mA until 4.3 V. After 4.3 V was maintained for one hour, discharge was performed at 6 mA until 3.0 V. This was one charge and discharge cycle, and three cycles in total of charge and discharge were performed to stabilize the buttery.

### <Measurement of Initial Resistance>

After the initial charge and discharge, the battery was charged at 25°C and 6 mA until 4.3 V, and a resistance value was directly measured by impedance measurement.

In each of Tables 1 to 8, the initial resistance value of Comparative Example using the comparative nonaqueous electrolytic solution to which neither the compound represented by General Formula (1) nor a comparative compound had been added (Comparative Example 1-1 in Table 1, Comparative Example 2-1 in Table 2, Comparative Example 3-1 in Table 3, Comparative Example 4-1 in Table 4, Comparative Example 5-1 in Table 5, Comparative Example 6-1 in Table 6, Comparative Example 7-1 in Table 7, Comparative Example 8-1 in Table 8) was shown as 100, and an evaluation result of the initial resistance in each of Examples and Comparative Examples was shown as a relative value.

As is clear from Tables 1 to 8, it is found that a nonaqueous electrolytic solution battery using a nonaqueous electrolytic solution including a compound represented by General Formula (1) of the present disclosure has low initial resistance and thus is excellent.

### INDUSTRIAL APPLICABILITY

According to the present disclosure, a nonaqueous electrolytic solution battery and a nonaqueous electrolytic solution having a low initial resistance value can be provided. A compound that can be suitably used in the above nonaqueous electrolytic solution can be provided.

Although the present disclosure has been described in detail and with reference to specific examples, it will be apparent to those skilled in the art that various changes and modifications can be made without departing from the spirit and scope of the present disclosure.

The present application is based on a Japanese Patent Application No. 2021-009171 filed on January 22, 2021, the contents of which are incorporated herein by reference.

## Claims

1. A nonaqueous electrolytic solution, comprising:
a compound represented by the following General Formula (1);
a solute; and
a nonaqueous organic solvent, 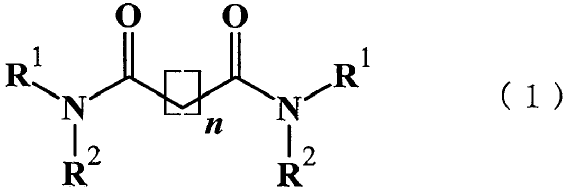
wherein in the General Formula (1), R¹'s each independently represent PO(R_{f})₂ or SO₂R_{f}, R_{f}'s each independently represent a fluorine atom, a linear alkyl group having 1 to 4 carbon atoms, or a branched alkyl group having 3 to 4 carbon atoms, an oxygen atom may be included between a carbon atom-carbon atom bond in the alkyl group, and any hydrogen atom of the alkyl group may be substituted with a fluorine atom,
R²'s each independently represent a hydrogen atom, a lithium ion, a sodium ion, a potassium ion, a linear alkyl group having 1 to 12 carbon atoms, or a branched alkyl group having 3 to 12 carbon atoms, an oxygen atom may be included between a carbon atom-carbon atom bond in the alkyl group, any hydrogen atom of the alkyl group may be substituted with a fluorine atom, the alkyl group may contain an unsaturated bond, and when R² represents a lithium ion, a sodium ion, or a potassium ion, a bond between a nitrogen atom and R² in the General Formula (1) represents an ionic bond, and
n represents an integer of 0 to 3.

2. The nonaqueous electrolytic solution according to claim 1, wherein
R¹'s in the General Formula (1) are each independently POF₂ or SO₂F.

3. The nonaqueous electrolytic solution according to claim 1 or 2, wherein
both R¹'s in the General Formula (1) are SO₂F.

4. The nonaqueous electrolytic solution according to any one of claims 1 to 3, wherein
R²'s in the General Formula (1) each independently represent a hydrogen atom, a lithium ion, a sodium ion, a linear alkyl group having 1 to 4 carbon atoms, or a branched alkyl group having 3 to 4 carbon atoms.

5. The nonaqueous electrolytic solution according to any one of claims 1 to 4, wherein
the compound represented by the General Formula (1) is a compound represented by the following Formula (1a),

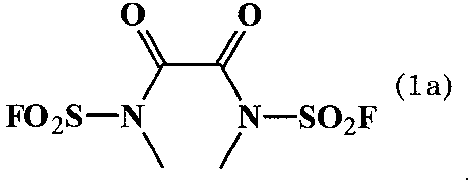

6. The nonaqueous electrolytic solution according to any one of claims 1 to 5, wherein
the nonaqueous organic solvent contains at least one selected from the group consisting of cyclic carbonate and chain carbonate.

7. The nonaqueous electrolytic solution according to claim 6, wherein
the cyclic carbonate is at least one selected from the group consisting of ethylene carbonate, propylene carbonate, and fluoroethylene carbonate, and the chain carbonate is at least one selected from the group consisting of ethyl methyl carbonate, dimethyl carbonate, diethyl carbonate, and methyl propyl carbonate.

8. The nonaqueous electrolytic solution according to any one of claims 1 to 7, wherein
a content of the compound represented by the General Formula (1) with respect to a total amount of the compound represented by the General Formula (1), the solute, and the nonaqueous organic solvent is 0.001% by mass to 10.0% by mass.

9. The nonaqueous electrolytic solution according to any one of claims 1 to 8, further comprising:
at least one selected from vinylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, and lithium (difluorophosphoryl)(fluorosulfonyl)imide in an amount of 0.01% by mass to 5.0% by mass with respect to a total amount of the nonaqueous electrolytic solution.

10. The nonaqueous electrolytic solution according to any one of claims 1 to 9, further comprising:
at least one compound selected from the group consisting of compounds represented by the following General Formulas (2) to (3) in an amount of 0.01% by mass to 5.0% by mass with respect to a total amount of the nonaqueous electrolytic solution, 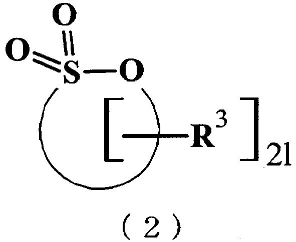
wherein the General Formula (2) represent a compound having a cyclic skeleton composed of 4 to 7 atoms selected from any of carbon atoms, oxygen atoms, and sulfur atoms, and has at least one adjacent sulfonyl group and oxygen atom in the cyclic skeleton, the cyclic skeleton may contain an unsaturated bond, a state of the sulfur atom forming the cyclic skeleton is selected from any of S, SO, and SO₂, l represents the number of carbon atoms contained in the cyclic skeleton, R³'s are bonded to the carbon atom forming the cyclic skeleton and each independently represent a hydrogen atom, a fluorine atom, or an aliphatic hydrocarbon group having 1 to 6 carbon atoms, and the aliphatic hydrocarbon group may contain at least one of an oxygen atom and an unsaturated bond, and any hydrogen atom in the aliphatic hydrocarbon group may be substituted with a fluorine atom, when the carbon atoms forming the cyclic skeleton are adjacent to each other, these carbon atoms may be bonded to form an aromatic ring, and a hydrocarbon group having 1 to 4 carbon atoms may be bonded to the aromatic ring, the hydrocarbon group bonded to the aromatic ring may contain at least one of an oxygen atom and an unsaturated bond, and any hydrogen atom of the hydrocarbon group may be substituted with a fluorine atom, and 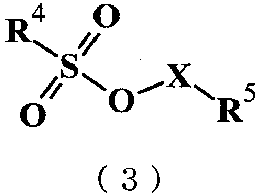
in the General Formula (3), X represents a sulfonyl group or a ketone group, and R⁴ and R⁵ are each independently selected from aliphatic hydrocarbon groups having 1 to 10 carbon atoms, at least one selected from the group consisting of a fluorine atom, an oxygen atom, an unsaturated bond, and an ester bond may be present in the aliphatic hydrocarbon group.

11. A nonaqueous electrolytic solution battery, comprising:
a positive electrode;
a negative electrode; and
the nonaqueous electrolytic solution according to any one of claims 1 to 10.

12. A compound represented by the following General Formula (1), 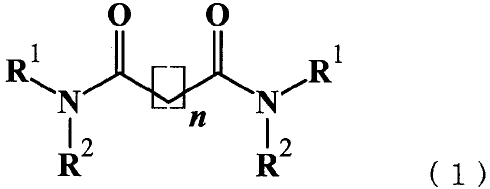
wherein in the General Formula (1), R¹'s each independently represent POF₂ or SO₂F,
R²'s each independently represent a hydrogen atom, a lithium ion, a sodium ion, a potassium ion, a linear alkyl group having 1 to 12 carbon atoms, or a branched alkyl group having 3 to 12 carbon atoms, and an oxygen atom may be included between a carbon atom-carbon atom bond in the alkyl group, any hydrogen atom of the alkyl group may be substituted with a fluorine atom, the alkyl group may contain an unsaturated bond, and when R² represents a lithium ion, a sodium ion, or a potassium ion, a bond between a nitrogen atom and R² in the General Formula (1) represents an ionic bond, and
n represents an integer of 0 to 3.

13. A compound represented by the following Formula (1a),
